# EUROPEAN PATENT APPLICATION

(11) **EP 2 232 986 A2**
(43) Date of publication of application: **29.09.2010**
(21) Application number: 10158039.7
(22) Date of filing: 26.03.2010
(51) Int. Cl.: A01K 67/033, A01N 63/00, A01P 7/02

(54) **Mite composition, the rearing thereof and methods for application of the composition in biological control systems**

(30) Priority: 26.03.2009 ES 200900826
(71) Applicant: Agrobio S.L., 04745 El Viso (La Mojonera) - Almeria (ES)
(72) Inventor: Vila Rifà, Enrique, 04745 El Viso (La Mojonera) - Almería (ES)
(74) Representative: De Hoop, Eric

(57) **Abstract**

The present invention relates to a mite composition, the rearing thereof and methods for application of the composition in biological control systems. The invention provides a mite composition comprising:
- a viable population of a phytoseiid predatory mite species selected from the group consisting of *Amblyseius montdorensis* (Schicha), *Typlodromalus lailae* (Schicha) and *Amblyseius largoensis* (Muma), preferably *Amblyseius montdorensis,*
- a viable factitious host population comprising at least one astigmatid mite species,
- an artificial rearing substrate which also acts as a carrier during distribution,
- wherein the astigmatid mite is selected from the family of the Acaridae, such as *Acarus siro,* Linnaeus, 1758.

The composition is used for application in commercial crops, especially tomatoes for pest control.

## Description

In general, this invention relates to a mite composition, the rearing thereof, and the application on biological control programmes.

The introduction of biological programmes using arthropod species as the controlling agents has in the second half of the 20^{th} century shown a steady increase in interest from growers of a wide range of crops, in particular those grown in the warmer geographic regions such as the Mediterranean basin. So much so that this practice has grown from amateur led schemes by individual growers to large commercial developments where international companies produce beneficial agents year round in large quantities, which are supplied to countries on a world wide basis.

Accordingly, the practice of biological control is growing steadily, year by year, as government health and safety regulators, wholesale companies and their retail customers, increase their demand for chemically clean produce. The benefits accruing to growers and consumers resulting from a corresponding decrease in the use of chemical pesticides is obvious and very considerable.

The first and most common application was the development of the commercial rearing of the phytoseiid *Neoseiulus cucumeris* (formerly assigned to the genus *Amblyseius*), and to a very limited extent *Amblyseius mackensie,* using the astigmatid species *Acarus farris,* Oudemans, as the host. The main target was the control of the western Flower Thrip.

More recently, because of the increasing problems with whitefly infestations, especially in Spain, the phytoseiid *Amblyseius swirskii* has been developed for commercial use, and is being sold in Spain by a number of biological control companies. It is used both against whiteflies and thrips for all but one crop, namely tomato.

Currently there is only one phytoseiid predator able to survive and develop on tomato plants. This is *Phytoseiulus persimilis,* a monophagus feeder, living entirely on spider mites, and used extensively as a biological control agent against this pest.

Apart from spider mites the remaining main pests of tomatoes are whiteflies, two species of tarsonemid mites and certain moth species. The latter may be controlled using viral or bacterial biocides, whilst parasitic hymenoptera are used against white-flies. However, biological control systems have yet to be successfully developed against the two tarsonemid mite species.

Moreover, in recent years a further serious problem has arisen from the invasion of thrips into this crop, especially in the warmer climes of the Mediterranean basin.

Currently there is no satisfactory biological control programme for this pest when it is present in a tomato crop, and as thrip infestations become more common there is a strong possibility that the biological programme will be abandoned and that chemicals will be used again instead.

Some success has been achieved from the application of mirid bugs, particularly *Macrolophus caliginosus* and *Mesidiocoris tenuis.* These bugs, however, have two disadvantages: They are omnivorous, eating beneficial as well as pest species, and feeding on tomato plant, then causing, sometimes, necrotic rings on stems and flowers and punctures in fruits.

An ideal solution would be to include the thrips and whiteflies predators *Amblyseius cucumeris* and *Amblyseius swirskii* into the biological programme, but this is not possible for tomato.

During the evolution of the family Solenaceae, from which the present day tomato evolved, the plants have developed a defensive mechanism against its ancestral pest, the spider mites. This defence has been achieved through the development of glandular hairs on both leaf surfaces. Hair density inhibits free movement of the spider mites, as do the secretions from these hairs, which may also have a toxic effect (Rodriquez *et al*, 1972). Unfortunately, this mechanism is also effective against the currently commercially available phytoseiid predatory species used against thrips and whiteflies.

In the document EP 1686849 A1, a mite composition is described comprising a population of the phytoseiid predatory mite *Amblyseius swirskii* and a population of a factitious host selected from the family of the Carpoglyphidae, which can be used to rear the said phytoseiid predatory mite and to release the phytoseiid predator into the crops. Also, in another aspect the invention described in EP 1686849 A1 relates to a system for rearing the phytoseiid predatory mite *Amblyseius swirskii,* to the use of said composition and a method to apply biological control on crops using such composition.

WO 2008104807 (A2) describes a composition comprising a population of predatory mites and a host population of the family Suidasiidae or Chortoglyphidae, where the predatory mite is the phytoseid mite *Amblyseius swirskii.*

According to the state of the art and the exposed problems, it would be advisable to get a phytoseiid predator which could survive on tomato plants and which would not have the disadvantages explained before.

The present inventors found three phytoseiid predatory mites which appear to be able to tolerate life on tomato plants. When combining any of these phytoseiid predatory mites with a specific factitious host a mite composition is obtained showing very good results in pest control on tomato plants.

Accordingly, in a first embodiment the present invention provides a mite composition comprising:
- a viable population of a phytoseiid predatory mite species selected from the group consisting of *Amblyseius montdorensis* (Schicha), *Typlodromalus lailae* (Schicha) and *Amblyseius largoensis* (Muma), preferably *Amblyseius montdorensis,*
- a viable factitious host population comprising at least one astigmatid mite species,
- an artificial rearing substrate which also acts as a carrier during distribution,
- wherein the astigmatid mite is selected from the family of the Acaridae, such as *Acarus siro,* Linnaeus, 1758.

Preferably, the relation between the number of individual predators and factitious host ranges from 1:10 to 1:100.

A second embodiment of this invention is the rearing system of said predatory mites and the use of the composition in applications of biological control.

The phytoseiid predatory mite species is selected from the group consisting of *Amblyseius montdorensis* (Schicha), *Typlodromalus lailae* (Schicha) (= *Typhlodromalus limonicus*) and *Amblyseius largoensis* (Muma), preferably *Amblyseius montdorensis.* These mite species are known to be efficient predators of various pests.

The factitious host is selected from the family of the Acaridae and is most preferably *Acarus siro.* In the embodiment of using *Acarus siro* as a food source for *Amblyseius montdorensis, Typlodromalus lailae* and *Amblyseius largoensis,* it can be described as a 'true' factitious host since it has naturally a very restricted habitat, being confined to food stores and domestic dwellings feeding upon a wide variety of whole grain cereals and their farinaceous by-products, as well as being a serious pest of stored cheese (Griffiths, 1966). It is not naturally found on plants, and has never been known to damage plants in any stage of their growth. It does not occur in any sylvan habitat occupied by any other astigmatid species nor such habitats associated with predaceous phytoseiids which are employed or are projected for use in biological control.

Although it is common to find in the prior art literature, published up to the middle of the 20^{th} century, many records of it being found in sylvan natural habitats, these are without exception misidentifications. Griffiths, 1966, showed that *Acarus siro, sensu stricto,* L.,1758 actually represents a complex of three species, two of which he identified or described as *Acarus farris,* Oudemans, and *Acarus immobilis,* Griffiths, and both of which are ephemeral inhabitants of stored foods, especially newly harvested grain. They do not survive the physical conditions of storage, leaving *Acarus siro,* L. as the sole storage species within the complex, probably because the relative humidity requirements for *Acarus siro* are lower than those required by the other two species of the complex.

Thus, in the embodiment of the use of *Acarus siro* in this invention it is the only true food storage species of the complex, and therefore can be defined as a true factitious host to phytoseiid predator species.

Prior art documentation on *Acarus siro* contains exhaustive data on its biology, ecology and habitat distribution, enabling the selection of ideal conditions for its commercial rearing (Cunnington, 1965 & 1985; Griffiths, 1964 & 1966, and Hughes, 1976). For example, Cunnington, in his treatise on the oviposition and fecudity of A. *siro* showed that under optimum physical conditions females averaged 435 eggs, with a maximum of 850, and a mean daily rate of egg production at 20-25 degrees centigrade and 90% RH, averaging 28 eggs per female over six days.

Griffiths, (1964) showed that on a diet of yeast powder development time from egg to egg was just 9.0 days.

Trials on the intrinsic rate of development of A. *montdorensis* on the factitious host A. *siro* have been completed (see example 1). These results prove that the combination of A. *montdorensis* with the factitious host *Acarus siro* allows a reliable production of the predator.

Unlike one of the commonly used astigmatid species, *Tyrophagus putrescentiae, A. siro* does not actively move far if they accidentally escape from a rearing system. This is a considerable advantage over the commonly used T. *putrescentiae* which are notorious for contaminating other commercial systems within a commercial enterprise. Their gentle activity also means they do not disturb the predator females in their egg-laying pursuits.

Thus, for the commercial producer of biological control agents *Acarus siro* is an ideal factitious host to *Amblyseius montdorensis* (Schicha), *Typhlodromalus lailae* Schicha and *Amblyseius largoensis* (Muma), particularly, to *Amblyseius montdorensis.*

In one embodiment of the invention about the rearing of the predatory species, *Amblyseius montdorensis* (Schicha), *Typhlodromalus lailae* (Schicha) and *Amblyseius largoensis* (Muma), preferably *Amblyseius montdorensis,* with A. *siro* as the food, it can be reared in convenient sized containers, providing there are arrangements for oxygen exchange. For this purpose, and according to another embodiment of the invention, the food supply and rearing substrate (carrier) is made up of an artificial mixture of yeast powder and various particulate sized pieces of vermiculite, plus a certain proportion of sterile dried fruit particles.

According to the last embodiment of the present invention, the food supply and rearing substrate, because of the free flowing inert nature, provides application advantages, relative to the nature of the crop, crop densities, plant configurations and management practices. For example the predator can be applied directly on to individual plants using appropriate sized biodegradable shaker bottles. In another embodiment it can be provided to the grower in large bulk quantities for transfer to the tanks of back-pack blower machines for easy distribution over large beds of ornamental plants. In some sophisticated glasshouses, these tanks are attached to machines which are automatically moved on overhead gantries to distribute the predator into wide beds of dense crops such as chrysanthemums.

The inert material vermiculite plus sterile dried particles of a dried fruit product precludes the problems arising when bran flakes are included in the medium. Bran, which is nowadays the most common carrier and food used to introduce phytoseiid species into crops, is naturally infected with storage fungi such as *Penicillium* and *Aspergillus* species so that when they become wet after application to crops such as ornamental and stemmed flowers mould growth occurs which disfigures the leaves and makes the plant less attractive to the retail customer. With this new diet for the rearing system, according to the present invention, growers of these crops can now safely use the sachet product, which is a clear advantage against the other previously known rearing food systems.

In a secondment of this host diet, the inventors have shown a further advantage of using such a certain percentage of fine, sterile particles of a dried fruit material: it enhances the well being and therefore strong development of the factitious host populations.

The said advantages are obtained because these particles help to maintain the medium at the correct moisture content which is especially important when the predator prey mixture is contained in paper sachets hanging in the crop. It overcomes also earlier problems where soon after planting when sachets were attached to a very young crop the absence of leaf cover and the proximity of low lying heating pipes caused the contents to dry out very quickly. The astigmatid mites, which breathe throughout the skin, are very sensitive to humidity changes; Already Cunnington (1965) showed that below relative humidities of 70 % many species of astigmatid mites die. Preferably, the composition according to the present invention is kept at 20-25°C and relative humidity between 75-85% in the rearing system.

Relative to pest attack this time in the plant's life is critical, and one in which it needs a full compliment of beneficial agents. A sachet content which clearly assists the factitious host to survive these conditions and provides food to the predators, more robust, is an innovative success.

As a result, with this new diet the producers of these crops will be able now to use safely the product supplied on sachets.

In a third embodiment of the invention, the mite composition of the present invention is used to provide a new large source of phytoseiid predators for the control of pest species of thrips on tomatoes, which without this invention may well encourage pesticide usage and the elimination of an on going viable biological control programme. Preferably, the combination of the present invention is used on crops infested by the trips species *Thrips tabaci* and *Frankiniella occidentalis,* and other local species, as well as 2 species of the Tarsonemid family, *Aculops lycopersici* and the broad mite *Polyphagotarsonemus latus.* The tomato plants can be grown in glasshouses, or plastic houses, netted shelters, or under field conditions.

Where feasible use may be extended to cover other crops such as peppers, cucumbers, melons, watermelons, eggplant, zucchini, green beans and drupe fruits, like strawberry, raspberry, etc (which may also encompass field grown crops).

According to the third embodiment of the present invention, small quantities of the active substrate, about 50-100 gms are placed in sachets made of a breathable paper, supplied with small exit holes from which the predators can emerge and small hooks so that the sachets can be hung at required intervals on individual plants (Sampson, 1998).

In a special embodiment, depending on the ratio of predator to prey within the sachet, the sachet can remain active from one to four weeks. For a short release time the system is termed a -"Quick Release Sachet", for the four week period it is termed a "Controlled Release System" The former is used if the pest has recently invaded the crop before the predator can be introduced. The latter can be introduced into the crop well in advanced of the expected time of arrival of a pest, thereby giving instant means of protection.

### Example 1. The Technical Trials carried out to estimate the rearing potential of Amblyseius montdorensis when feeding upon its factitious host Acarus siro.

The objective is to determine the potential intrinsic rate of increase and certain life history parameters of a population of A. *montdorensis* when reared upon the factitious host A. *siro.*

### Methodology

Both predator and prey individuals used for the trials were randomly selected from stock cultures of the two species. The trials were conducted in an atmosphere of 25°C and 75-80% Relative Humidity (RH).

### Oviposition trials

To evaluate the fecundity of A. *montdorensis* when feeding A. *siro,* very small populations, as described below, were contained in a series of individual small cells cut from a strip of black plastic sheet. Five circular holes, diameter circa 10-12mm were drilled at intervals along a black strip about 40mm width, 4mm depth and about 80mm in length. The undersides of the cells were sealed with a strip of breathable paper, attached to the strip with non-toxic wood glue. Small squares of thin clear plastic (Plexiglass^{®}), each with a central hole some 3 mm diameter were used as a seal for each cell. Each trial used four strips, totalling 20 females, i.e. 5 females per strip.

On day zero of a trial a small population of the factitious host was placed in the first cell of each strip, sufficient to maintain five young female predators for three days.

Five such females were picked out of a culture and transferred on a camel hair paint brush into cell No. 1, via the central hole, which was then sealed with a square of glass, the whole held together by two clips attached on either side of the main strip.

After a period of two days the females were collected and released into the next cell in line, where some factitious hosts were also added. This process was repeated until the last of the four cells had been filled. Egg production per five females per two day interval was recorded. (See the Results paragraph, Table 1.)

### Rate of Development trials

Using the small cells described above, three females per cell were allowed to lay eggs for 48 hours. After 24 hours females were collected and placed on the next cell 24 hours more. They were then removed, leaving eggs and a very small amount of food material in each cell. Only eggs from the second day were used for the trial.

At an average of some 2.4 eggs per female per day, each cell at the start contained about 6 to 8 eggs.

To evaluate the rate of development, cells were examined at 24 hour intervals until all individuals had emerged as adults. At each examination each active mite was identified to stage and recorded. When all individuals had passed through a particular stage the records were examined and an average rate of development of this stage calculated.

Eggs from a total of 20 females were followed through to adult emergence. Mortality was not calculated. (See the Results paragraph, Table 2.)

### Intrinsic Rate of Increase trials

To evaluate the Intrinsic Rate of Increase, small plastic boxes were used, about 11.5 cm length, 8.5 cm width and 4.5 cm depth. A hole on the lid was made and it was sealed with breathable paper, to avoid exits and to allow air exchange.

Using the quartering and coning technique for estimating population density (individuals per kg. of culture) a supply of predators and their prey, representing a density of 30,000 predators per kg. was divided into lots and each lot placed in an individual cell, held under the same conditions as described above, at a ratio of predator: prey sufficient to permit ten days development. This is an adequate time scale for the trial since most commercial production systems run on a 7-10 cycle.

At the end of the trial time the populations were reassessed and the intrinsic rate of increase estimated. Eight replications were used (see the Results paragraph, Table 3).

### RESULTS

**Table 1. Ovipositon rate for Amblyseius montdorensis fed upon the factitious host Acarus siro.**

| Days between counts | No. of females per 4 cells strips | Total eggs laid per count | Eggs per female per day |
|---|---|---|---|
| 2 | 20 | 64 | 1.6 |
| 2 | 20 | 100 | 2.5 |
| 2 | 20 | 116 | 2.9 |
| 2 | 20 | 108 | 2.7 |
| 2 | 20 | 92 | 2.3 |
| Total 10 | 20 | 480 | 2.4 |

**Table 2. Rate of development of the life stages of Amblyseius montdorensis (egg hatch to adult emergence) when fed upon the factitious host Acarus siro.**

| Average time for development (days) | | | |
|---|---|---|---|
| Larva | Protonymph | Tritonymph | Total |
| 1.7 - 2.0 | 2.0 - 2.5 | 3.0 - 3.5 | 6.7 - 8.0 |

**Table 3. Intrinsic Rate of Development of Amblyseius montdorensis when reared on the factitious host Acarus siro, (Start population per replicate of 30,000 nymph and adult predators).**

| Intrinsic Rate of Increase over 10 days (Averages of eight replicates) | | |
|---|---|---|
| Minimum | Maximum | Mean |
| x 1.6 | x 4.1 | x 3.35 |

The results of the Intrinsic Rate of Increase of *Amblyseius montdorensis* on the factitious host *Acarus siro* prove that this combination has multiple advantages compared with the other combinations known from the state of art, using any other astigmatid host, both considering the easiness of rearing on *Acarus siro,* as well as the data about rearing and development. Thus, for the commercial producer of biological control agents, *Acarus siro* is an ideal factitious host for rearing *Amblyseius montdorensis,* and other phytoseiid species, specially *Typlodromalus lailae* and *Amblyseius largoensis.*

### References

Cunnington, A.M. 1965. Physical limits for complete development of the Grain Mite, Acarus siro (Acarina, Acaridae), in relation to its world distribution. J. appl. Ecol. 2, pp.295-306.
Cunnington, A.M. 1985. Factors affecting oviposition and fecundity in the Grain Mite, Acarus siro L., (Acarina: Acaridiae), especially temperature and humidity. Experimental and Applied Acarology, 1, pp. 327-44.
Griffiths, D.A. 1964. A revision of the genus Acarus (Acaridiae, Acarina), Bull. Brit. Mus. (nat. Hist.) (Zool.), 11 pp. 413-464.
Hughes, A.M. 1976. The mites of stored food and houses. Ministry of Agriculture Fisheries and Food, Her Majest's Stationery Office, London, pp. 400.
Rodriguez, J. G., Knavel, D.E., & Aina, O.J. 1972. Studies in the resistance of tomatoes to mites. J. Econ. Ent., 65, (1), pp. 50-3.
Sampson, C. 1998, The commercial development of an Amblyseius cucumeris controlled release method for the control of Frankiniella ocidentalis in protected crops. The Brighton Conference - Pests & Diseases, 5B-4, pp. 409-16.

## Claims

1. A mite composition comprising:
- a viable population of a phytoseiid predatory mite species selected from the group consisting of *Amblyseius montdorensis* (Schicha), *Typlodromalus lailae* (Schicha) and *Amblyseius largoensis* (Muma), preferably *Amblyseius montdorensis,*
- a viable factitious host population comprising at least one astigmatid mite species,
- an artificial rearing substrate which also acts as a carrier during distribution,
- wherein the astigmatid mite is selected from the family of the Acaridae, such as *Acarus siro,* Linnaeus, 1758.

2. The composition according to claim 1, wherein the rearing substrate is made up of an artificial mixture of yeast powder and various particulate sized pieces of vermiculite, plus a proportion of sterile dried fruit particles.

3. Composition according to claims 1 and 2 wherein the ratio of the individual predators and the factitious host ranges from about 1:10 to 1:100.

4. A method for rearing the phytoseiid predatory species *Amblyseius montdorensis, Typlodromalus lailae* and *Amblyseius largoensis,* preferably *Amblyseius montdorensis,* comprising providing a combination of mites according to any of claims 1-3, and allowing the predatory population to feed the individuals of the said factitious host.

5. Method according to claim 4, wherein the composition is maintained at 20-25°C and 75-85% relative humidity.

6. Method according to claims 4 or 5, wherein the composition according to claims 1-3 is maintained in a three dimensional bulk form, suitable for the adequate development of the predator and its prey, providing there are arrangements for oxygen exchange.

7. Method according to claim 6, wherein the composition is contained in a rearing container having such a construction as to permit exit of the mobile life stages of the predator phytoseiid mite species.

8. Use of the composition according to claims 1-3 for application in commercial crops for pest control.

9. Use according to claim 8, wherein the crop pest is selected from the thrip species *Thrips tabaci* and *Frankiniella occidentalis,* and other local species, and in addition two mite species from the family Tarsonemidae, namely the tomato Russet Mite, *Aculops lycopersicae* (Massee), and the Broad Mite, *Polyphagotarsonemus latus* (Banks).

10. Use according to claims 8-9, wherein the crop to be treated is tomato, grown in glasshouses, or plastic houses, netted shelters, or under field conditions.

11. Use according to claim 8, wherein the composition according to claims 1-3 is distributed on the crop using a rearing container having such a construction as to permit exit of the mobile life stages of the predator phytoseiid mite species, suitable for direct sprinkler application over crops.

12. Use according to claim 8, wherein the composition according to claims 1-3 is distributed on the crop using a rearing container having such a construction as to permit exit of the mobile life stages of the predator phytoseiid mite species, suitable for hanging directly upon individual plants.
